(19) Europäisches Patentamt
European Patent Office
Office européen des brevets

(11) EP 0 671 382 B1

(12) EUROPÄISCHE PATENTSCHRIFT

(45) Veröffentlichungstag und Bekanntmachung des Hinweises auf die Patenterteilung:
**14.10.1998 Patentblatt 1998/42**

(51) Int. Cl.[6]: **C07C 211/15**, C07C 209/70, C07C 229/12, C07C 309/13

(21) Anmeldenummer: **95103150.9**

(22) Anmeldetag: **06.03.1995**

(54) **Fluorhaltige Carboxylbetaine und Alkylsulfobetaine sowie deren Mischungen mit gesättigten Fluoralkyaminen**

Fluorinated carboxybetaines and alkylsulfobetaines as well as the mixtures thereof with saturated fluoroalkylamines

Carboxybetaines et alkylsulfobetaines fluorées ainsi que leurs mélanges avec les fluoroalkylamines saturées

(84) Benannte Vertragsstaaten:
**BE CH DE FR GB IT LI NL**

(30) Priorität: **09.03.1994 US 208004**

(43) Veröffentlichungstag der Anmeldung:
**13.09.1995 Patentblatt 1995/37**

(73) Patentinhaber: **Clariant GmbH**
**65929 Frankfurt am Main (DE)**

(72) Erfinder:
- **Prossel, Günter, Dr.**
  **D-84508 Burgkirchen (DE)**
- **Knaup, Wolfgang, Dr.**
  **D-84508 Burgkirchen (DE)**
- **Wehowsky, Frank, Dr.**
  **D-84508 Burgkirchen (DE)**

(56) Entgegenhaltungen:
**EP-A- 0 057 925**
**EP-A- 0 296 046**
**EP-A- 0 296 935**
**EP-A- 0 311 473**
**US-A- 4 059 629**
**US-A- 4 183 367**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 671 382 B1

## Beschreibung

Die Erfindung betrifft fluorhaltige Carboxyalkylbetaine und Sulfobetaine. Die Erfindung betrifft ferner Mischungen dieser Tenside mit gesättigten Fluoralkylaminen.

Gesättigte und ungesättigte Fluoralkylamine sind wertvolle Ausgangsprodukte zur Herstellung von kationischen oder amphoteren Fluoralkylverbindungen wie Fluoralkylbetainen, die vielseitig einsetzbare Tenside darstellen, zum Beispiel als Mittel zur Erniedrigung der Oberflächenspannung des Wassers (Wasser/Luft) oder der Grenzflächenspannung von Wasser/Kohlenwasserstoff-Systemen.

So werden in US-A 3 535 381 ungesättigte Fluoralkylamine der Formel $R_f$-CF=CH-$CH_2$-NZ beschrieben, worin $R_f$ einen Perfluoralkylrest und NZ den Rest eines primären oder sekundären Amins bedeuten. Sie werden durch Umsetzung von einem Fluoralkylethylen mit einem primären oder sekundären Amin hergestellt, wie die folgende Reaktionsgleichung zeigt

$$R_f\text{-}CF_2\text{-}CH{=}CH_2 + 2HNZ \rightarrow R_f\text{-}CF{=}CH\text{-}CH_2\text{-}NZ + [ZNH_2]^+F^-$$

Ihre quaternären Ammoniumverbindungen sollen gute Wirksamkeit in der Erniedrigung der Oberflächenspannung des Wassers aufweisen.

In US-A 4 059 629 werden gesättigte Fluoralkylamine der Formel $R_f$-$(CH_2)_m$-NZ, worin $R_f$ und NZ die angegebenen Bedeutungen haben und m 2 oder 4 ist, sowie Mischungen bestehend aus diesen gesättigten und den oben erwähnten ungesättigten Fluoralkylaminen als Mittel zur Absenkung der Oberflächenspannung des Wassers empfohlen.

In US-A 4 183 367 werden schließlich Carboxyalkylbetaine mit einer gesättigten Fluoralkylgruppe und in US-A 4 430 272 Alkylsulfobetaine mit einer ungesättigten Fluoralkylgruppe als wirksame Tenside beschrieben.

Mit der vorliegenden Erfindung werden nun neue fluorhaltige Carboxyalkylbetaine und Alkylsulfobetaine sowie deren Mischungen mit gesättigten Fluoralkylaminen und Mischungen mit den neuen Fluorbetainen zur Verfügung gestellt.

Die gesättigten Fluoralkylamine entsprechen der nachstehenden Formel (1)

$$\begin{array}{l} \qquad\qquad\qquad\qquad\qquad\qquad\quad R^1 \\ \qquad\qquad\qquad\qquad\qquad\qquad\quad / \\ C_nF_{2n+1}\text{-}CFH\text{-}CH_2\text{-}CH_2\text{-}N \\ \qquad\qquad\qquad\qquad\qquad\qquad\quad \backslash \\ \qquad\qquad\qquad\qquad\qquad\qquad\quad R^2 \end{array} \qquad (1)$$

worin bedeuten n eine ganze Zahl von 3 bis 17, vorzugsweise 5 bis 13, und $R^1$ und $R^2$ $C_1$ bis $C_4$-Alkyl, $C_1$ bis $C_4$-Hydroxyalkyl oder Wasserstoff, mit der Maßgabe, daß nur einer der beiden Substituenten, $R^1$ und $R^2$, Wasserstoff ist.

Der Perfluoralkylrest $C_nF_{2n+1}$ kann geradkettig oder verzweigt sein, wobei geradkettig bevorzugt ist. Im Falle einer verzweigten Perfluoralkylgruppe ist die endverzweigte bevorzugt. Als Beispiele seien genannt $C_3F_7$, $C_5F_{11}$, $C_7F_{15}$, $C_9F_{19}$ und $C_{11}F_{23}$ sowie Gemische von Perfluoralkylhomologen mit der angegebenen Bedeutung von n, zum Beispiel $C_5F_{11}$ bis $C_{11}F_{23}$-Gemische ($C_5F_{11}/C_{11}F_{23}$). $R^1$ und $R^2$, die vorzugsweise Methyl oder Ethyl sind, können gleich oder verschieden sein. Die Hydroxyalkylgruppe ist vorzugsweise -$CH_2CH_2$-OH.

Die gesättigten Fluoralkylamine der Formel (1) werden hergestellt durch Hydrierung von Fluoralkenylaminen der nachstehenden Formel (1a)

$$\begin{array}{l} \qquad\qquad\qquad\qquad\qquad\quad R^1 \\ \qquad\qquad\qquad\qquad\qquad\quad / \\ C_nF_{2n+1}\text{-}CF{=}CH\text{-}CH_2\text{-}N \\ \qquad\qquad\qquad\qquad\qquad\quad \backslash \\ \qquad\qquad\qquad\qquad\qquad\quad R^2 \end{array} \qquad (1a)$$

worin für n, $R^1$, $R^2$ und den Perfluoralkylrest die angegebenen Bedeutungen gelten.

Die ungesättigten Fluoralkylamine (1a) sind bekannt und in der eingangs genannten US-A 3,535,381 ausführlich beschrieben.

In EP-A-0 296 046 werden mit Übergangsmetallen katalysierte Hydrierungen von Substanzen der Formel (1a)

beschrieben, die im wesentlichen gesättigte Amine der Formel (1) ergeben. In EP-A-0 296 935 werden in Beispiel 11 ähnliche Hydrierungen einer - allerdings verunreinigten - Verbindung der Formel (1a) beschrieben. Schließlich werden in der vorliegenden Erfindung fünf repräsentative Beispiele für eine nahezu optimale Hydrierung von Verbindungen der Formel (1a) mit Edelmetallkatalysatoren beschrieben, wobei Verbindungen der Formel (1) in hoher Reinheit und Ausbeute erhalten werden.

Die so hergestellten gesättigten Fluoralkylamine mit der speziellen γ-CFH-Einheit sind bei Raumtemperatur flüssig, ausgenommen jene mit einer besonders langen Perfluoralkylgruppe. Sie sind thermostabiler und pH-stabiler als die ungesättigten Ausgangs-Fluoralkylamine und damit auch über einen längeren Zeitraum lagerbeständig. Sie stellen vorteilhafte Ausgangsverbindungen (Zwischenprodukte) dar zur Herstellung von neuen Carboxyalkylbetainen und Alkylsulfobetainen und von neuen Mischungen enthaltend die gesättigten Fluoralkylamine.

Die erfindungsgemäßen Carboxyalkylbetaine entsprechen der nachstehenden Formel (2)

$$C_nF_{2n+1}\text{-CFH-}CH_2\text{-}CH_2\text{-}\overset{R^1}{\underset{R^2}{N^+}}\text{-}(CH_2)_a\text{-}COO^- \qquad (2)$$

worin a 1, 2, 3 oder 4 ist, vorzugsweise 1, und für n, $R^1$, $R^2$ und den Perfluoralkylrest die bei Formel (1) angegebenen Bedeutungen gelten.

Die erfindungsgemäßen Carboxyalkylbetaine werden durch Carboxyalkylierung von Verbindungen der Formel (1) hergestellt. Als Betainisierungsmittel oder Alkylierungsmittel wird eine Halogencarbonsäure der nachstehenden Formel (2a)

$$X\text{-}(CH_2)_a\text{-}COOH \qquad (2a)$$

worin X ein Halogen ist, vorzugsweise Cl oder Br, und a die angegebene Bedeutung hat,
eingesetzt, oder ein Salz davon, vorzugsweise ein Alkalimetallsalz, oder ein $C_1$ bis $C_4$-Alkylester dieser Halogencarbonsäure. Im Falle der Verwendung eines Halogencarbonsäureesters ist das Alkylierungsprodukt klarerweise einer Verseifung zu unterwerfen, um die Verbindung der Formel (2) zu erhalten. Die Betainisierung wird bevorzugt bei einer Temperatur von 60 bis 100 °C, vorzugsweise 70 bis 95 °C, und in Gegenwart eines Lösungsmittels aus der Gruppe der niedrigen Alkanole oder Wasser oder Mischungen von Wasser und niedrigen Alkanolen wie Methanol, Ethanol, Propanol und/oder Isopropanol (vorzugsweise im Volumenverhältnis von 1 Teil Wasser zu 10 bis 30 Teilen Alkanol) durchgeführt. Das Lösungsmittel wird in einer solchen Menge eingesetzt, daß eine 20 bis 70gew.%ige, vorzugsweise 30 bis 50gew.%ige, Fluoralkylamin-Lösung vorliegt. Zur Betainisierung des tertiären Fluoralkylamins ist stöchiometrisch 1 mol Halogencarbonsäure, Esterverbindung oder Halogencarbonsäuresalz pro mol Fluoralkylamin erforderlich. Zur Erreichung einer möglichst vollständigen Betainisierung werden deshalb 1 bis 1,1 mol, vorzugsweise 1 bis 1,03 mol, Betainisierungsmittel pro mol tertiäres Fluoralkylamin eingesetzt. Der pH-Wert der Reaktionsmischung liegt im allgemeinen bei 5 bis 8, vorzugsweise bei 6,5 bis 7,5. Die bei Atmosphärendruck oder mehr oder weniger leichtem Überdruck verlaufende Umsetzung wird im allgemeinen so lange aufrechterhalten, bis das gesamte tertiäre Fluoralkylamin betainisiert ist. Alkyliert man mit Halogencarbonsäure wird die gebildete Halogenwasserstoffsäure vorzugsweise mit einem Alkalimetallhydroxid zu Alkalimetallhalogenid gebunden. Das Alkalimetallhydroxid (NaOH oder KOH) wird vorzugsweise in Form einer 30 bis 60gew.%igen wäßrigen Lösung eingesetzt. Eine Aufarbeitung der so erhaltenen Lösung von γ-CFH-Carboxyalkylbetainen der Formel (2), zum Beispiel durch Abdestillieren des Lösungsmittels und Gewinnung fester Carboxyalkylbetaine, ist oft gar nicht erforderlich, da auch die Lösungen vielfach verwendet werden können.

Die erfindungsgemäßen Alkylsulfobetaine entsprechen der nachstehenden Formel (3)

$$C_nF_{2n+1}\text{-CFH-}CH_2\text{-}CH_2\text{-}\overset{R^1}{\underset{R^2}{N}}\text{-}(CH_2)_b\text{-}SO_3^- \qquad (3)$$

worin b 1, 2, 3 oder 4 ist, vorzugsweise 3 oder 4, und für n, $R^1$, $R^2$ und den Perfluoralkylrest die bei Formel (1)

angegebenen Bedeutungen gelten.

Die erfindungsgemäßen Alkylsulfobetaine werden durch Sulfoalkylierung von Verbindungen der Formel (1) hergestellt. Als Sulfoalkylierungsmittel wird ein Sulton der nachstehenden Formel (3a)

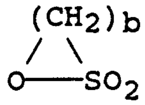

(3a)

worin b die angegebene Bedeutung hat, eingesetzt, vorzugsweise Propansulton oder Butansulton. Die Sulfoalkylierung wird in Gegenwart eines organischen Lösungsmittels durchgeführt, das gegenüber den Reaktionspartnern inert ist. Geeignete Lösungsmittel sind beispielsweise Methanol, Ethanol, Butylglykol, Butyldiglykol oder Aceton. Die zweckmäßige Reaktionstemperatur liegt im Bereich von 50 bis 100 °C, die Reaktion erfolgt unter im wesentlichen drucklosen Bedingungen. Bei diesen Temperaturen dauert die Sulfoalkylierung zwischen 1 und 10 Stunden. Die Sultone sollten zweckmäßig nicht über den stöchiometrisch erforderlichen Anteil hinaus eingesetzt werden, da sie toxisch sind. Nach Beendigung der Sulfoalkylierung können die γ-CFH-Alkylsulfobetaine der Formel (3) durch Abdestillieren des Lösungsmittels in fester Form gewonnen werden. Für viele Anwendungszwecke sind aber auch die anfallenden Lösungen der neuen Sulfobetaine einsetzbar.

Die erfindungsgemäßen Carboxyalkylbetaine und Sulfobetaine mit dem besonderen Merkmal -CFH- im Fluoralkylrest weisen außergewöhnliche Tensideigenschaften auf. Sie bewirken eine unerwartet starke Erniedrigung der Oberflächenspannung von Wasser/Luft-Systemen und der Grenzflächenspannung von Wasser/Kohlenwasserstoff-Systemen, wobei oft nur sehr kleine Einsatzmengen erforderlich sind.

Die Mischungen von gesättigten Fluoralkylaminverbindungen bestehen im wesentlichen aus

A) 60 bis 90 Gew.-%, vorzugsweise 75 bis 85 Gew.-%, von mindestens einem gesättigten Fluoralkylamin der angegebenen Formel (1) und

B) 10 bis 40 Gew.-%, vorzugsweise 15 bis 25 Gew.-%, von mindestens einem gesättigten Fluoralkylamin der nachstehenden Formel (4)

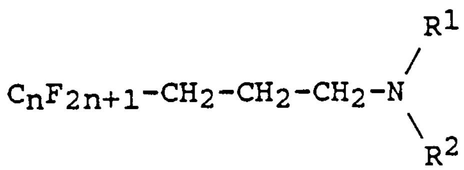

(4)

worin n, $R^1$, $R^2$ und der Perfluoralkylrest die bei Formel (1) angegebenen Bedeutungen haben,

Gewichtsprozente bezogen auf das Gewicht der Mischung.

Die Mischungen aus gesättigten Fluoralkylaminen der Formeln (1) und (4) werden hergestellt durch Hydrierung von Fluoralkenylaminen der oben angegebenen Formel (1a) in Gegenwart eines Katalysators aus der Gruppe Eisen, Kobalt und Nickel und bei einer Temperatur von 0 bis 50 °C, vorzugsweise 15 bis 30 °C. Mit der Kombination aus einem Hydrierkatalysator aus der Eisengruppe und einer speziellen Hydriertemperatur wird erreicht, daß die Hydrierung von Verbindungen der Formel (1a) zu den beschriebenen Mischungen bestehend im wesentlichen aus den gesättigten Fluoralkylaminen der Formeln (1) und (4) in hoher Ausbeute führt. Ihre Bildung dürfte aus zwei verschiedenen Reaktionsmechanismen resultieren. Die Verbindungen der Formel (1) werden offensichtlich durch Hydrierung der -CF=CH-Gruppe in (1a) zu $-CFH-CH_2-$ gebildet und die Verbindungen der Formel (4) dürften durch weitere Hydrierung von $-CFH-CH_2-$ unter Freisetzung von HF entstehen, was die folgende Reaktionsgleichung veranschaulichen soll:

$$-CFH-CH_2- + H_2 \rightarrow -CH_2-CH_2- + HF$$

Nach Abschluß der in flüssiger Phase durchgeführten Hydrierung liegt die angestrebte Mischung aus Verbindungen der Formeln (1) und (4) vor. Falls die Abtrennung des eingesetzten Katalysators und die Gewinnung einer katalysatorfreien Mischung gewünscht wird, kann dies zum Beispiel durch Dekantierung oder Filtrierung erreicht werden. Zur weiteren Reinigung des Produktes kann man ein oder mehrmals mit Wasser waschen und gegebenenfalls noch destillieren, vorzugsweise in Form einer Wasserdampfdestillation. Es wurde gefunden, daß die beim Waschen auftretenden

Gele durch Alkalisieren auf pH 8 bis 10 zerstört werden können. Die erfindungsgemäßen Mischungen von Verbindungen der Formeln (1) und (4) werden in hoher Ausbeute und Reinheit erhalten. Sie sind bei Raumtemperatur flüssig, ausgenommen jene mit einer besonders langen Perfluoralkylgruppe. Sie sind thermostabiler und pH-stabiler als die ungesättigten Ausgangs-Fluoralkylamine und damit auch über einen längeren Zeitraum lagerbeständig. Sie stellen vorteilhafte Ausgangsverbindungen (Zwischenprodukte) dar, da sie durch Betainisierung sehr wirksame Tensid-Mischungen von Carboxyalkylbetainen und/oder Sulfobetainen liefern.

Die erfindungsgemäßen Carboxyalkylbetain-Mischungen bestehen demnach im wesentlich aus

A) 60 bis 90 Gew.-%, vorzugsweise 75 bis 85 Gew.-%, von mindestens einem Carboxyalkylbetain der angegebenen Formel (2) und
B) 10 bis 40 Gew.-%, vorzugsweise 15 bis 25 Gew.-%, von mindestens einem Carboxyalkylbetain der nachstehenden Formel (5)

$$C_nF_{2n+1}-CH_2-CH_2-CH_2-\overset{\displaystyle R^1}{\underset{\displaystyle R^2}{\overset{|}{\underset{|}{N}}}}-(CH_2)_a-COO^- \qquad (5)$$

worin a, n, $R^1$, $R^2$ und der Perfluoralkylrest die bei Formel (2) angegebenen Bedeutungen haben.

Die erfindungsgemäßen Sulfobetain-Mischungen bestehen im wesentlichen aus

A) 60 bis 90 Gew.-%, vorzugsweise 75 bis 85 Gew.-%, von mindestens einem Alkylsulfobetain der angegebenen Formel (3) und
B) 10 bis 40 Gew.-%, vorzugsweise 15 bis 25 Gew.-%, von mindestens einem Alkylsulfobetain der nachstehenden Formel (6)

$$C_nF_{2n+1}-CH_2-CH_2-CH_2-\overset{\displaystyle R^1}{\underset{\displaystyle R^2}{\overset{|}{\underset{|}{N}}}}-(CH_2)_b-SO_3^- \qquad (6)$$

worin b, n, $R^1$, $R^2$ und der Perfluoralkylrest die bei Formel (3) angegebenen Bedeutungen haben.

Diese erfindungsgemäßen Tensidmischungen werden durch Carboxyalkylierung oder Sulfoalkylierung der oben beschriebenen Mischungen aus Verbindungen der Formeln (1) und (4) hergestellt, wobei jeweils in der gleichen Art und Weise carboxyalkyliert und sulfoalkyliert wird, wie oben für die Verbindungen der Formel (1) beschrieben. Die erhaltenen Carboxyalkylbetain-Mischungen und Sulfoalkylbetain-Mischungen weisen eine überraschend hohe Wirksamkeit in der Erniedrigung der Oberflächenspannung von Wasser/Luft-Systemen und der Grenzflächenspannung von Wasser/Kohlenwasserstoff-Systemen, wobei häufig nur sehr kleine Einsatzmengen erforderlich sind.

Die Erfindung wird nun anhand von Beispielen noch näher erläutert.

Das folgende Beispiel 1 betrifft die erfindungsgemäßen Verbindungen der Formel (2):

Beispiel 1

In einer 500-ml-Rührapparatur (ausgestattet mit Kühler, Tropftrichter und Thermometer) wurden 75 g (0,15 mol) $C_nF_{2n+1}$-CHF-$(CH_2)_2$-$N(CH_3)_2$, 19,5 g Natriumchloracetat (98%ig, 0,16 mol) und 126 g Ethanol/$H_2O$ (20 : 1) vorgelegt. Die Reaktionsmischung wurde 30 Stunden lang unter Rühren am Rückfluß (das sind etwa 80 °C) gehalten. Im Laufe der Reaktion wurden 2,4 g 30gew.%iges wäßriges NaOH zugegeben. Nach beendeter Reaktion wurde zur weitgehenden Entfernung von NaCl über eine auf 60 bis 70 °C beheizte Druckfilterpresse filtriert. Das Filtrat wurde am Rotationsverdampfer zur Trockne eingedampft. Es wurden 82 g (das ist eine Ausbeute von 95 % der Theorie) Carboxymethylbetain (mit einem Restgehalt an NaCl von 2 Gew.-%) der nachstehenden Formel erhalten:

$C_nF_{2n+1}$-CHF-$(CH_2)_2$-$N^+(CH_3)_2$-$CH_2CO_2^-$

Charakterisierung durch 1H-NMR ($CD_3OD$):

| | |
|---|---|
| γ-CHF: | 5,44 ppm (1H, d, m) |
| β-$CH_2$: | 2,45 ppm (2H, m) |
| α-$CH_2$: | 4,00 ppm (2H, m) |
| $N(CH_3)_2$: | 3,36 ppm (6H, s) |
| $CH_2$-$CO_2^-$: | 3,95 ppm (2H, s) |

Der Perfluoralkylrest $C_nF_{2n+1}$ ist ein Gemisch perfluorierter $C_5$-, $C_7$-, $C_9$- und $C_{11}$-Alkyle mit folgender Zusammensetzung in gaschromatographisch bestimmten Flächenprozentwerten: $C_5 : C_7 : C_9 : C_{11} = 24 : 59 : 16 : 1$

Das erfindungsgemäße Carboxymethylbetain wurde bezüglich seiner Wirksamkeit in der Erniedrigung der Oberflächenspannung des Wassers (mN/m) getestet, und zwar unter Verwendung verschiedener Konzentrationen (in Gewichtsprozent) bei jeweils 80 °C. Die Ergebnisse sind nachstehend zusammengefaßt:

| Gew.-% | mN/m |
|---|---|
| 0,1 | 14,2 |
| 0,04 | 14,3 |
| 0,02 | 14,5 |
| 0,01 | 15,1 |
| 0,005 | 17,4 |

Das folgende Beispiel 2 betrifft die erfindungsgemäßen Verbindungen der Formel (3):

Beispiel 2

In einer 500-ml-Rührapparatur (ausgestattet mit Kühler, Tropftrichter und Thermometer) wurden 100 g (0,216 mol) $C_nF_{2n+1}$-CHF-$(CH_2)_2$-$N(CH_3)_2$ und 146 g Monoethylenglykolmonobutylether (Butylglykol) vorgelegt und auf 60 °C erhitzt. Der erhitzten Mischung wurden 26,5 g (0,217 mol) Propansulton zudosiert, worauf die Reaktionsmischung 1 Stunde lang bei 60 °C und anschließend weitere 5 Stunden bei 105 bis 110 °C gerührt wurde. Zur Aufarbeitung wurde das Reaktionsgemisch mit 146 g Wasser versetzt und 2 Stunden lang bei 95 °C gerührt (Hydrolyse von überschüssigem Propansulton). Das Sulfobetain der Formel $C_nF_{2n+1}$-CHF-$(CH_2)_2$-$N^+(CH_3)_2$-$(CH_2)_3SO_3^-$ wurde in einer Ausbeute von 86 % der Theorie erhalten.

Für den Perfluoralkylrest $C_nF_{2n+1}$ gilt das im Beispiel 6 Gesagte.

Das erfindungsgemäße Alkylsulfobetain wurde bezüglich seiner Wirksamkeit in der Erniedrigung der Oberflächenspannung des Wassers (mN/m) getestet, und zwar unter Verwendung verschiedener Konzentrationen (in Gewichtsprozent) bei jeweils 60 °C. Die Ergebnisse sind nachstehend zusammengefaßt:

| Gew.-% | mN/m |
|---|---|
| 0,1 | 15,7 |
| 0,04 | 16,1 |
| 0,02 | 16,4 |
| 0,01 | 16,7 |
| 0,005 | 17,8 |

Die folgenden Beispiele 3 bis 6 betreffen die erfindungsgemäßen Mischungen aus Verbindungen der Formel (2) und Formel (5):

Beispiel 3

Herstellung einer Mischung bestehend im wesentlichen aus 80 Gew.-% $C_nF_{2n+1}$-CHF-$(CH_2)_2$-$N^+(CH_3)_2$-$CH_2CO_2^-$ und 20 Gew.-% $C_nF_{2n+1}$-$(CH_2)_3$-$N^+(CH_3)_2$-$CH_2CO_2^-$.

In einer 4-l-Rührapparatur (mit Kühler, Tropftrichter und Thermometer ausgestattet) wurden 554,3 g (1,0 mol) von einer Mischung aus 80 Gew.-% $C_nF_{2n+1}$-CHF-$(CH_2)_2$-$N(CH_3)_2$ und 20 Gew.-% $C_nF_{2n+1}$-$(CH_2)_3$-$N(CH_3)_2$, 130,8 g Natriumchloracetat (98%ig, 1,1 mol), 1022 g iso-Propanol und 100 g Wasser vorgelegt. Die Reaktionsmischung wurde 34 Stunden lang unter Rückfluß (etwa 80 °C) und Rühren erhitzt. Im Laufe der Reaktion wurden 2,4 g NaOH als 30gew.%ige wäßrige Lösung zugegeben. Nach beendeter Reaktion wurde zur weitgehenden Entfernung von NaCl über eine auf 60 bis 70 °C beheizte Druckfilterpresse filtriert. Das Filtrat wurde am Rotationsdampfer zur Trockne eingeengt. Es wurden 612 g (das ist eine Ausbeute von 94 % der Theorie) der angegebenen erfindungsgemäßen Carboxymethylbetain-Mischung (mit einem Restgehalt von 3,2 Gew.-% NaCl und 3,1 Gew.-% Na-Glykolat) erhalten. Charakterisierung durch $^1$H-NMR ($CD_3OD$):

Betain-Komponente der Formel

$$C_nF_{2n+1}\text{-CHF-}(CH_2)_2\text{-}N^+(CH_3)_2\text{-}CH_2CO_2^-:$$

| | |
|---|---|
| γ-CHF: | 5,44 ppm (1H, d, m) |
| β-$CH_2$: | 2,45 ppm (2H, m) |
| α-$CH_2$: | 4,00 ppm (2H, m) |
| $N(CH_3)_2$: | 3,36 ppm (6H, s) |
| $CH_2$-$CO_2^-$: | 3,95 ppm (2H, s) |

Betain-Komponente der Formel

$$C_nF_{2n+1}\text{-}(CH_2)_3\text{-}N^+(CH_3)_2\text{-}CH_2CO_2^-:$$

| | |
|---|---|
| γ-$CH_2$: | 2,45 ppm (2H, m) |
| β-$CH_2$: | 2,15 ppm (2H, m) |
| α-$CH_2$: | 3,76 ppm (2H, m) |
| $N(CH_3)_2$: | 3,32 ppm (6H, s) |
| $CH_2$-$CO_2^-$: | 3,95 ppm (2H, s) |

Der Perfluoralkylrest $C_nF_{2n+1}$ ist ein Gemisch perfluorierter $C_5$-, $C_7$-, $C_9$- und $C_{11}$-Alkyle mit folgender Zusammensetzung in gaschromatographisch bestimmten Flächenprozentwerten: $C_5 : C_7 : C_9 : C_{11}$ = 4 : 59 : 36 : 1

Die erfindungsgemäße Carboxymethylbetain-Mischung wurde bezüglich ihrer Wirksamkeit in der Erniedrigung der Oberflächenspannung des Wassers (mN/m) getestet, und zwar unter Verwendung verschiedener Konzentrationen (in Gewichtsprozent) bei jeweils 35 °C. Die Ergebnisse sind nachstehend zusammengefaßt:

| Gew.-% | mN/m |
|---|---|
| 0,1 | 18,1 |
| 0,05 | 19,1 |
| 0,02 | 19,3 |
| 0,01 | 19,5 |

Beispiel 4

Herstellung einer Mischung bestehend im wesentlichen aus 80 Gew.-% $C_nF_{2n+1}$-CHF-$(CH_2)_2$-$N^+(CH_3)_2CH_2CO_2^-$ und 20 Gew.-% $C_nF_{2n+1}$-$(CH_2)_3$-$N^+(CH_3)_2$-$CH_2CO_2^-$.

$C_nF_{2n+1}$ hat die folgende Zusammensetzung:
$C_5 : C_7 : C_9 : C_{11}$ = 27 : 56 : 15 : 2

In einer 4-l-Rührapparatur wurde eine Mischung von 483,3 g $C_nF_{2n+1}$-CHF-$(CH_2)_2$-$N(CH_3)_2$ und $C_nF_{2n+1}$-$(CH_2)_3$-$N(CH_3)_2$ im Gewichtsverhältnis von 80 : 20 (1,0 mol), 130,8 g Natriumchloracetat (98%ig, 1,1 mol), 773 g Ethanol und

39 g Wasser vorgelegt. Die Reaktionsmischung wurde 30 Stunden unter Rückfluß (etwa 80 °C) und Rühren erhitzt. Im Laufe der Reaktion wurden 2,2 g NaOH als 30%ige wäßrige Lösung zugegeben. Nach beendeter Reaktion wurde zur weitgehenden Entfernung von NaCl über eine auf 60 bis 70 °C beheizte Druckfilterpresse filtriert. Man erhielt 1295 g (96 % der Theorie) einer 40%igen Lösung der erfindungsgemäßen Carboxymethylbetain-Mischung (mit einem Restgehalt an NaCl von 1,9 Gew.-% und einem Restamingehalt von 0,8 Gew.-%, jeweils bezogen auf das Trockenprodukt). Charakterisierung des Betains wie in Beispiel 3.

Die erfindungsgemäße Carboxymethylbetain-Mischung wurde bezüglich ihrer Wirksamkeit in der Erniedrigung der Oberflächenspannung des Wassers (mN/m) getestet, und zwar unter Verwendung verschiedener Konzentrationen (in Gewichtsprozent) bei jeweils 80 °C. Die Ergebnisse sind nachstehend zusammengefaßt:

| Gew.-% | mN/m |
|---|---|
| 0,1 | 14,2 |
| 0,04 | 14,2 |
| 0,02 | 14,9 |
| 0,01 | 14,9 |
| 0,005 | 14,9 |

Die erfindungsgemäße Mischung aus gesättigten Carboxymethylbetainen wurde auch bezüglich Thermostabilität getestet und mit ungesättigtem Carboxymethylbetain der Formel $C_nF_{2n+1}$-$CF=CH$-$CH_2$-$N^+(CH_3)_2CH_2CO_2^-$ verglichen, wobei $C_nF_{2n+1}$ die angegebene Bedeutung hat. Von den beiden Testprodukten wurden jeweils 2gew.%ige Lösungen in Wasser/Isopropanol (im Volumenverhältnis von 8 : 1) als Lösemittel hergestellt. Der pH-Wert der Lösungen wurde mit Diethanolamin auf jeweils 8 eingestellt. Von den beiden Lösungen wurde auch der Fluorid- Gehalt bestimmt, der bei < 1 ppm lag. Die beiden Testlösungen hatten also einen pH-Wert von 8 und einen Fluorid-Gehalt von < 1 ppm. Diese Ausgangslösungen wurden auf 65 °C erhitzt und bei dieser Temperatur gehalten. Nach 4 und 10 Tagen wurde der pH-Wert und der Fluorid-Gehalt bestimmt. Das Ergebnis ist nachstehend zusammengefaßt und zeigt, daß das erfindungsgemäße Produkt wesentlich thermostabiler ist, da sowohl pH-Wert als auch Fluorid-Gehalt während der gesamten Testzeit im Gegensatz zum Vergleichsprodukt unverändert bleiben:

| | 4 Tage | | 10 Tage | |
|---|---|---|---|---|
| | pH-Wert | Fluorid | pH-Wert | Fluorid |
| erfindungsgemäßes Produkt | 8 | < 1 ppm | 8 | < 1 ppm |
| Vergleichsprodukt | 6,1 | 16 ppm | 5,6 | 24 ppm |

Beispiel 5

Dieses Beispiel soll zeigen, daß man die erfindungsgemäße Carboxymethylbetain-Mischung auch ausgehend von Monochloressigsäure herstellen kann.

In einer 1-l-Rührapparatur wurden 320 g Ethanol, 6 g Wasser und 17,6 g NaOH-Prills (0,44 mol) vorgelegt, worauf so lange bei 50 °C gerührt wurde, bis kein festes NaOH mehr vorhanden war. Jetzt wurden bei 50 °C langsam (während circa 30 Minuten) 52 g 80gew.%ige Monochloressigsäure (0,44 mol) zugetropft, wobei Natriumchloracetat in Form eines feinen Pulvers teilweise ausfiel. Nun wurden 193 g (0,4 mol) von der im Beispiel 4 eingesetzten Mischung $C_nF_{2n-1}$-$CHF$-$(CH_2)_2$-$N(CH_3)_2$ und $C_nF_{2n+1}$-$(CH_2)_3$-$N(CH_3)_2$ im Gewichtsverhältnis von 80 : 20 zugegeben, worauf die Reaktionsmischung 29 Stunden lang bei 80 °C gerührt wurde. Im Laufe der Reaktion wurden 10,6 g 30gew.%ige wäßrige NaOH-Lösung zugegeben. Die Aufarbeitung nach beendeter Reaktion erfolgte wie im Beispiel 11. Man erhielt 524 g (95 % der Theorie) einer 39%igen Lösung der erfindungsgemäßen Carboxyethylbetain-Mischung (mit einem Restgehalt an NaCl von 2,1 Gew.-% und einem Restamingehalt von 1,2 Gew.-%, jeweils bezogen auf Trockenprodukt). Charakterisierung des Betains wie im Beispiel 3.

Beispiel 6

Dieses Beispiel soll zeigen, daß man die erfindungsgemäße Carboxymethylbetain-Mischung auch mit Chloressigsäuremethylester als Carboxymethylierungsmittel herstellen kann.

Herstellung einer Mischung bestehend im wesentlichen aus
80 Gew.-% $C_5F_{11}$-CHF-$(CH_2)_2$-$N^+(CH_3)_2$-$CH_2CO_2^-$ und
20 Gew.-% $C_5F_{11}$-$(CH_2)_3$-$N^+(CH_3)_2$-$CH_2CO_2^-$.

In einer 500-ml-Rührapparatur wurden 112,4 g (0,3 mol) von einer Mischung aus 80 Gew.-% $C_5F_{11}$-CHF-$(CH_2)_2$-$N(CH_3)_2$ und 20 Gew.-% $C_5F_{11}$-$(CH_2)_3$-$N(CH_3)_2$, 36,7 g (0,3 mol) Cl-$CH_2$-$CO_2$-$C_2H_5$ und 160 g Ethanol 18 Stunden bei 80 °C gerührt. Anschließend wurden 24 g von einer 50gew.%igen wäßrigen NaOH-Lösung (0,3 mol) zugegeben und weitere 2,5 Stunden bei 80 °C gerührt. Das ausgefallene NaCl (14,7 g) wurde bei 60 bis 70 °C abfiltriert und das Filtrat am Rotationsverdampfer bis zur Trockne eingeengt. Es wurden 107,7 g (das ist eine Ausbeute von 81 % der Theorie) der angegebenen Carboxymethylbetain-Mischung (mit einem Restgehalt von NaCl von 2,5 Gew.-%) erhalten. Charakterisierung durch $^1$H-NMR wie im Beispiel 3.

Die folgenden Beispiele 7 und 8 betreffen die erfindungsgemäßen Mischungen aus Verbindungen der Formel (3) und Formel (6):

Beispiel 7

Herstellung einer Mischung bestehend im wesentlichen aus
80 Gew.-% $C_nF_{2n+1}$-CHF-$(CH_2)_2$-$N^+(CH_3)_2$-$(CH_2)_3SO_3^-$ und
20 Gew.-% $C_nF_{2n+1}$-$(CH_2)_3$-$N^+(CH_3)_2$-$(CH_2)_3SO_3^-$.

In einer 2-l-Rührapparatur (mit Kühler, Tropftrichter und Thermometer ausgestattet) wurden 300 g (0,624 mol) von einer Mischung aus 80 Gew.-% $C_nF_{2n+1}$-CHF-$(CH_2)_2$-$N(CH_3)_2$ und 20 Gew.-% $C_nF_{2n+1}$-$(CH_2)_3$-$N(CH_3)_2$ und 439 g Monoethylenglykolmonobutylether (Butylglykol) vorgelegt und auf 60 °C erhitzt. Der erhitzten Mischung wurden 76,9 g (0,630 mol) Propansulton zudosiert, worauf die Reaktionsmischung 40 Stunden lang bei 105 bis 110 °C gerührt wurde. Zur Aufarbeitung wurde das Reaktionsgemisch mit 438 g Wasser versetzt und 3 Stunden lang bei 90 °C gerührt (Hydrolyse von überschüssigem Propansulton). Die oben angegebene erfindungsgemäße Alkylsulfobetain-Mischung wurde in einer Ausbeute von 85 % der Theorie erhalten.

Für den Perfluoralkylrest $C_nF_{2n+1}$ gilt das im Beispiel 4 Gesagte.

Die erfindungsgemäße Sulfobetain-Mischung wurde bezüglich ihrer Wirksamkeit in der Erniedrigung der Oberflächenspannung des Wassers (mN/m) getestet, und zwar unter Verwendung verschiedener Konzentrationen (in Gewichtsprozent) bei jeweils Raumtemperatur (20 °C). Die Ergebnisse sind nachstehend zusammengefaßt:

| Gew.-% | mN/m |
|---|---|
| 0,1 | 20,4 |
| 0,05 | 20,7 |
| 0,02 | 21,7 |
| 0,01 | 21,7 |

Beispiel 8

Dieses Beispiel soll zeigen, daß man die erfindungsgemäße Sulfobetain-Mischung auch mit Butansulton als Sulfoalkylierungsmittel herstellen kann.

Herstellung einer Mischung bestehend im wesentlichen aus
80 Gew.-% $C_nF_{2n+1}$-CHF-$(CH_2)_2$-$N^+(CH_3)_2$-$(CH_2)_4SO_3^-$ und
20 Gew.-% $C_nF_{2n+1}$-$(CH_2)_3$-$N^+(CH_3)_2$-$(CH_2)_4SO_3^-$.

Für den Perfluoralkylrest $C_nF_{2n+1}$ gilt das im Beispiel 7 Gesagte.

In einer 250-ml-Rührapparatur wurden 48,1 g (0,1 mol) von einer Mischung aus 80 Gew.-% $C_nF_{2n+1}$-CHF-$(CH_2)_2$-$N(CH_3)_2$ und 20 Gew.-% $C_nF_{2n+1}$-$(CH_2)_3$-$N(CH_3)_2$ und 31 g Butylglykol vorgelegt und auf 110 °C erhitzt. Der erhitzten Mischung wurden 13,8 g (0,101 mol) Butansulfon zudosiert, worauf die Reaktionsmischung 16 Stunden lang bei etwa 120 °C gerührt wurde. Zur Aufarbeitung wurde das Reaktionsgemisch mit 31 g Wasser versetzt und 5 Stunden lang bei etwa 95 °C gerührt (Hydrolyse von überschüssigem Butansulton). Die erfindungsgemäße Sulfobetain-Mischung wurde

in einer Ausbeute von 85 % der Theorie erhalten.

**Patentansprüche**

1. Carboxyalkylbetaine der nachstehenden Formel (2)

$$C_nF_{2n+1}-CFH-CH_2-CH_2-\underset{\displaystyle R^2}{\overset{\displaystyle R^1}{\underset{|}{\overset{|}{N^+}}}}-(CH_2)_a-COO^- \qquad (2)$$

worin bedeuten a 1, 2, 3 oder 4, n eine ganze Zahl von 3 bis 17 und $R^1$ und $R^2$ $C_1$ bis $C_4$-Alkyl, $C_1$ bis $C_4$-Hydroxyalkyl oder Wasserstoff, mit der Maßgabe, daß nur einer der beiden Substituenten, $R^1$ und $R^2$, Wasserstoff ist.

2. Verfahren zur Herstellung der Carboxyalkylbetaine nach Anspruch 1, dadurch gekennzeichnet, daß man Fluoralkylamine der nachstehenden Formel (1)

$$C_nF_{2n+1}-CFH-CH_2-CH_2-N\begin{array}{l} \diagup R^1 \\ \diagdown R^2 \end{array} \qquad (1)$$

worin n, $R^1$ und $R^2$ die in Anspruch 1 angegebenen Bedeutungen haben,
carboxyalkyliert mit einer Halogencarbonsäure der nachstehenden Formel (2a)

$$X-(CH_2)_a-COOH \qquad (2a)$$

worin X ein Halogen ist und a die angegebene Bedeutung hat,
oder einem Salz davon oder einem $C_1$ bis $C_4$-Alkylester, wobei man im Falle der Verwendung des Halogencarbonsäureesters das Alkylierungsprodukt einer Verseifung unterwirft.

3. Verfahren zur Erniedrigung der Oberflächenspannung von Wasser/Luft-Systemen und der Grenzflächenspannung von Wasser/Kohlenwasserstoff-Systemen, dadurch gekennzeichnet, daß man dem System eine wirksame Menge von Carboxyalkylbetain nach Anspruch 1 zusetzt.

4. Alkylsulfobetaine der nachstehenden Formel (3)

$$C_nF_{2n+1}-CFH-CH_2-CH_2-\underset{\displaystyle R^2}{\overset{\displaystyle R^1}{\underset{|}{\overset{|}{N}}}}-(CH_2)_b-SO_3^- \qquad (3)$$

worin bedeuten b 1, 2, 3 oder 4, n eine ganze Zahl von 3 bis 17 und $R^1$ und $R^2$ $C_1$ bis $C_4$-Alkyl, $C_1$ bis $C_4$-Hydroxyalkyl oder Wasserstoff, mit der Maßgabe, daß nur einer der beiden Substituenten, $R^1$ und $R^2$, Wasserstoff ist.

5. Verfahren zur Herstellung der Alkylsulfobetaine nach Anspruch 4, dadurch gekennzeichnet, daß man Fluoralkylamine der nachstehenden Formel (1)

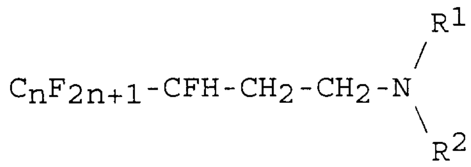

(1)

worin n, $R^1$ und $R^2$ die in Anspruch 4 angegebenen Bedeutungen haben, sulfoalkyliert mit einem Sulton der nachstehenden Formel (3a)

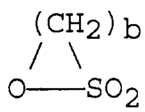

(3a)

worin b die in Anspruch 4 angegebene Bedeutung hat.

6. Verfahren zur Erniedrigung der Oberflächenspannung von Wasser/Luft-Systemen und der Grenzflächenspannung von Wasser/Kohlenwasserstoff-Systemen, dadurch gekennzeichnet, daß man dem System eine wirksame Menge von Alkylsulfobetain nach Anspruch 4 zusetzt.

7. Carboxyalkylbetain-Mischungen bestehend im wesentlichen aus

A) 60 bis 90 Gew.-% von mindestens einem Carboxyalkylbetain der nachstehenden Formel (2)

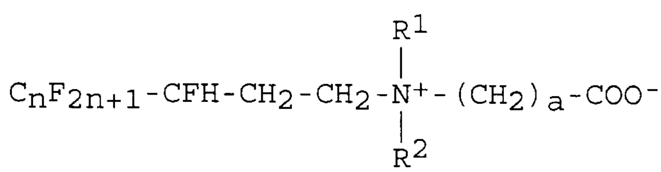

(2)

worin bedeuten a 1, 2, 3 oder 4, n eine ganze Zahl von 3 bis 17 und $R^1$ und $R^2$ $C_1$ bis $C_4$-Alkyl, $C_1$ bis $C_4$-Hydroxyalkyl oder Wasserstoff, mit der Maßgabe, daß nur einer der beiden Substituenten, $R^1$ und $R^2$, Wasserstoff ist, und

B) 10 bis 40 Gew.-% von mindestens einem Carboxyalkylbetain der nachstehenden Formel (5)

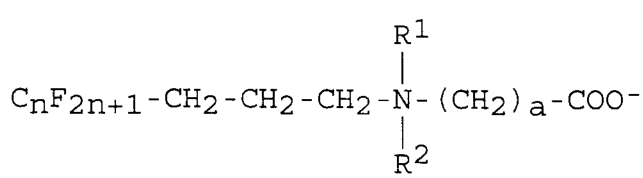

(5)

worin a, n, $R^1$ und $R^2$ die angegebenen Bedeutungen haben, Gewichtsprozente bezogen auf das Gewicht der Mischung.

8. Verfahren zur Herstellung der Mischungen nach Anspruch 7, dadurch gekennzeichnet, daß man Mischungen von Fluoralkylaminverbindungen bestehend im wesentlichen aus

A') 60 bis 90 Gew.-% von mindestens einem gesättigten Fluoralkylamin der nachstehenden Formel (1)

$$\begin{array}{ll} & R^1 \\ & / \\ C_nF_{2n+1}\text{-}CFH\text{-}CH_2\text{-}CH_2\text{-}N & \\ & \backslash \\ & R^2 \end{array} \qquad (1)$$

worin bedeuten n eine ganze Zahl von 3 bis 17 und $R^1$ und $R^2$ $C_1$ bis $C_4$-Alkyl, $C_1$ bis $C_4$-Hydroxyalkyl oder Wasserstoff, mit der Maßgabe, daß nur einer der beiden Substituenten, $R^1$ und $R^2$, Wasserstoff ist, und

B') 10 bis 40 Gew.-% von mindestens einem gesättigten Fluoralkylamin der nachstehenden Formel (4)

$$\begin{array}{ll} & R^1 \\ & / \\ C_nF_{2n+1}\text{-}CH_2\text{-}CH_2\text{-}CH_2\text{-}N & \\ & \backslash \\ & R^2 \end{array} \qquad (4)$$

worin n, $R^1$ und $R^2$ die angegebenen Bedeutungen haben, Gewichtsprozente bezogen auf das Gewicht der Mischung, carboxyalkyliert mit einer Halogencarbonsäure der nachstehenden Formel (2a)

$$X\text{-}(CH_2)_a\text{-}COOH \qquad (2a)$$

worin X ein Halogen ist und a die angegebene Bedeutung hat, oder einem Salz davon oder einem $C_1$ bis $C_4$-Alkylester, wobei man im Falle der Verwendung des Halogencarbonsäureesters das Alkylierungsprodukt einer Verseifung unterwirft.

**9.** Verfahren zur Erniedrigung der Oberflächenspannung von Wasser/Luft-Systemen und der Grenzflächenspannung von Wasser/Kohlenwasserstoff-Systemen, dadurch gekennzeichnet, daß man dem System eine wirksame Menge von Carboxyalkylbetain-Mischung nach Anspruch 7 zusetzt.

**10.** Sulfobetain-Mischungen bestehend im wesentlichen aus

A) 60 bis 90 Gew.-% von mindestens einem Alkylsulfobetain der nachstehenden Formel (3)

$$\begin{array}{c} R^1 \\ | \\ C_nF_{2n+1}\text{-}CFH\text{-}CH_2\text{-}CH_2\text{-}N\text{-}(CH_2)_b\text{-}SO_3^- \\ | \\ R^2 \end{array} \qquad (3)$$

worin bedeuten b 1, 2, 3 oder 4, n eine ganze Zahl von 3 bis 17 und $R^1$ und $R^2$ $C_1$ bis $C_4$-Alkyl, $C_1$ bis $C_4$-Hydroxyalkyl oder Wasserstoff, mit der Maßgabe, daß nur einer der beiden Substituenten, $R^1$ und $R^2$, Wasserstoff ist, und

B) 10 bis 40 Gew.-% von mindestens einem Alkylsulfobetain der nachstehenden Formel (6)

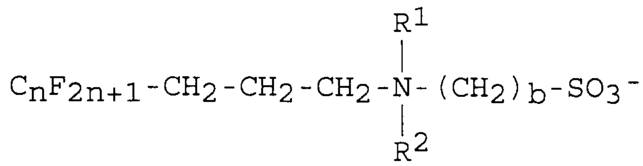 (6)

worin b, n, $R^1$ und $R^2$ die angegebenen Bedeutungen haben, Gewichtsprozente bezogen auf das Gewicht der Mischung.

**11.** Verfahren zur Herstellung der Mischungen nach Anspruch 10, dadurch gekennzeichnet, daß man Mischungen von Fluoralkylaminverbindungen bestehend im wesentlichen aus

A') 60 bis 90 Gew.-% von mindestens einem gesättigten Fluoralkylamin der nachstehenden Formel (1)

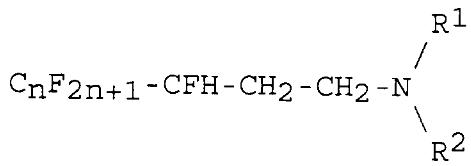 (1)

worin bedeuten n eine ganze Zahl von 3 bis 17 und $R^1$ und $R^2$ $C_1$ bis $C_4$-Alkyl, $C_1$ bis $C_4$-Hydroxyalkyl oder Wasserstoff, mit der Maßgabe, daß nur einer der beiden Substituenten, $R^1$ und $R^2$, Wasserstoff ist, und B') 10 bis 40 Gew.-% von mindestens einem gesättigten Fluoralkylamin der nachstehenden Formel (4)

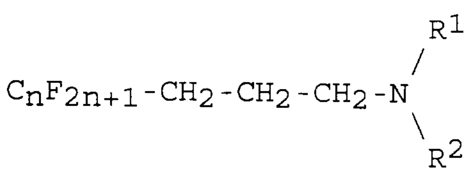 (4)

worin n, $R^1$ und $R^2$ die angegebenen Bedeutungen haben, Gewichtsprozente bezogen auf das Gewicht der Mischung, sulfoalkyliert mit einem Sulton der nachstehenden Formel (3a)

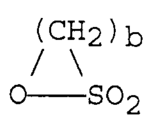 (3a)

worin b die angegebene Bedeutung hat.

**12.** Verfahren zur Erniedrigung der Oberflächenspannung von Wasser/Luft-Systemen und der Grenzflächenspannung von Wasser/Kohlenwasserstoff-Systemen, dadurch gekennzeichnet, daß man dem System eine wirksame Menge von Alkylsulfobetain-Mischung nach Anspruch 10 zusetzt.

## Claims

**1.** Carboxyalkylbetaines of the formula (2) below

$$C_nF_{2n+1}-CFH-CH_2-CH_2-\overset{\displaystyle R^1}{\underset{\displaystyle R^2}{\overset{|}{\underset{|}{N^+}}}}-(CH_2)_a-COO^- \qquad (2)$$

in which a is 1, 2, 3 or 4, n is an integer from 3 to 17 and $R^1$ and $R^2$ are $C_1$ to $C_4$ alkyl, $C_1$ to $C_4$ hydroxyalkyl or hydrogen, with the proviso that only one of the two substituents $R^1$ and $R^2$ is hydrogen.

2. Process for the preparation of the carboxyalkylbetaines according to claim 1, characterized in that fluoroalkylamines of the formula (1) below

$$C_nF_{2n+1}-CFH-CH_2-CH_2-N\begin{matrix}/R^1\\ \backslash R^2\end{matrix} \qquad (1)$$

in which n, $R^1$ and $R^2$ are as defined in claim 1, are carboxyalkylated with a halocarboxylic acid of the formula (2a) below

$$X\text{-}(CH_2)_a\text{-}COOH \qquad (2a)$$

in which X is a halogen and a is as defined, or a salt thereof or a $C_1$ to $C_4$ alkyl ester, the alkylation product being subjected to hydrolysis if the halocarboxylic ester is used.

3. Method of reducing the surface tension of water/air systems and the interfacial tension of water/hydrocarbon systems, characterized in that an effective quantity of carboxyalkylbetaine according to claim 1 is added to the system.

4. Alkylsulfobetaines of the formula (3) below

$$C_nF_{2n+1}-CFH-CH_2-CH_2-\overset{\displaystyle R^1}{\underset{\displaystyle R^2}{\overset{|}{\underset{|}{N}}}}-(CH_2)_b-SO_3^- \qquad (3)$$

in which b is 1, 2, 3 or 4, n is an integer from 3 to 17 and $R^1$ and $R^2$ are $C_1$ to $C_4$ alkyl, $C_1$ to $C_4$ hydroxyalkyl or hydrogen, with the proviso that only one of the two substituents $R^1$ and $R^2$ is hydrogen.

5. Process for the preparation of the alkylsulfobetaines according to claim 4, characterized in that fluoroalkylamines of the formula (1) below

$$C_nF_{2n+1}-CFH-CH_2-CH_2-N\begin{matrix}/R^1\\ \backslash R^2\end{matrix} \qquad (1)$$

in which n, $R^1$ and $R^2$ are as defined in claim 4, are sulfoalkylated with a sultone of the formula (3a) below

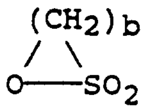

(3a)

in which b is as defined in claim 4.

6. Method of reducing the surface tension of water/air systems and the interfacial tension of water/hydrocarbon systems, characterized in that an effective quantity of alkylsulfobetaine according to claim 4 is added to the system.

7. Carboxyalkylbetaine mixtures essentially comprising

A) from 60 to 90 % by weight of at least one carboxyalkylbetaine of the formula (2) below

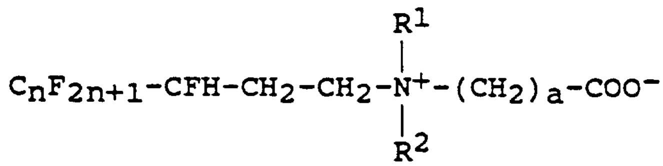

(2)

in which a is 1, 2, 3 or 4, n is an integer from 3 to 17 and $R^1$ and $R^2$ are $C_1$ to $C_4$ alkyl, $C_1$ to $C_4$ hydroxyalkyl or hydrogen, with the proviso that only one of the two substituents $R^1$ and $R^2$ is hydrogen, and
B) from 10 to 40 % by weight of at least one carboxyalkylbetaine of the formula (5) below

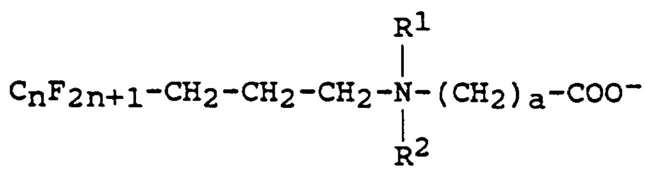

(5)

in which a, n, $R^1$ and $R^2$ are as defined, percentages by weight being based on the weight of the mixture.

8. Process for the preparation of the mixtures according to claim 7, characterized in that mixtures of fluoroalkylamine compounds essentially comprising

A') from 60 to 90 % by weight of at least one saturated fluoroalkylamine of the formula (1) below

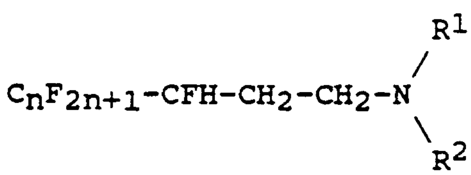

(1)

in which n is an integer from 3 to 17 and $R^1$ and $R^2$ are $C_1$ to $C_4$ alkyl, $C_1$ to $C_4$ hydroxyalkyl or hydrogen, with the proviso that only one of the two substituents $R^1$ and $R^2$ is hydrogen, and
B') from 10 to 40 % by weight of at least one saturated fluoroalkylamine of the formula (4) below

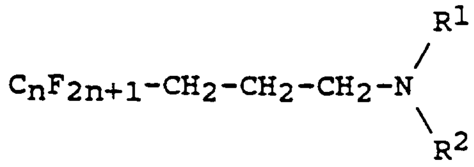 (4)

in which n, $R^1$ and $R^2$ are as defined, percentages by weight being based on the weight of the mixture, are carboxyalkylated with a halocarboxylic acid of the formula (2a) below

$$X\text{-}(CH_2)_a\text{-}COOH \qquad (2a)$$

in which X is a halogen and a is as defined, or a salt thereof or a $C_1$ to $C_4$ alkyl ester, the alkylation product being subjected to hydrolysis if the halocarboxylic ester is used.

**9.** Method of reducing the surface tension of water/ air systems and the interfacial tension of water/hydrocarbon systems, characterized in that an effective quantity of carboxyalkylbetaine mixture according to claim 7 is added to the system.

**10.** Sulfobetaine mixtures essentially comprising

A) from 60 to 90 % by weight of at least one alkylsulfobetaine of the formula (3) below

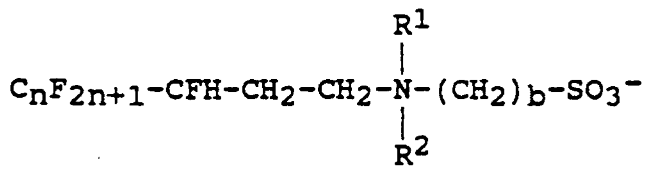 (3)

in which b is 1, 2, 3 or 4, n is an integer from 3 to 17 and $R^1$ and $R^2$ are $C_1$ to $C_4$ alkyl, $C_1$ to $C_4$ hydroxyalkyl or hydrogen, with the proviso that only one of the two substituents $R^1$ and $R^2$ is hydrogen, and
B) from 10 to 40 % by weight of at least one alkylsulfobetaine of the formula (6) below

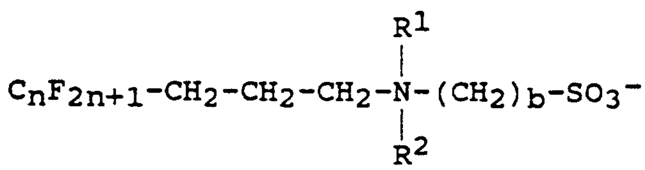 (6)

in which b, n, $R^1$ and $R^2$ are as defined, percentages by weight being based on the weight of the mixture.

**11.** Process for the preparation of the mixtures according to claim 10, characterized in that mixtures of fluoroalkylamine compounds essentially comprising

A') from 60 to 90 % by weight of at least one saturated fluoroalkylamine of the formula (1) below

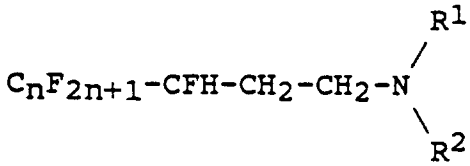

(1)

in which n is an integer from 3 to 17 and $R^1$ and $R^2$ are $C_1$ to $C_4$ alkyl, $C_1$ to $C_4$ hydroxyalkyl or hydrogen, with the proviso that only one of the two substituents $R^1$ and $R^2$ is hydrogen, and

B') from 10 to 40 % by weight of at least one saturated fluoroalkylamine of the formula (4) below

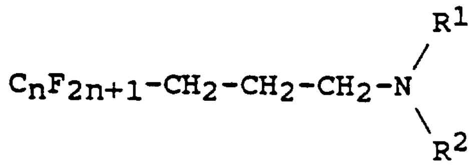

(4)

in which n, $R^1$ and $R^2$ are as defined, percentages by weight being based on the weight of the mixture, are sulfoalkylated with a sultone of the formula (3a) below

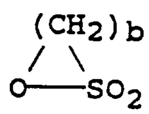

(3a)

in which b is as defined.

**12.** Method of reducing the surface tension of water/air systems and the interfacial tension of water/hydrocarbon systems, characterized in that an effective quantity of alkylsulfobetaine mixture according to claim 10 is added to the system.

**Revendications**

**1.** Carboxyalkylbétaïnes de formule (2) ci-après

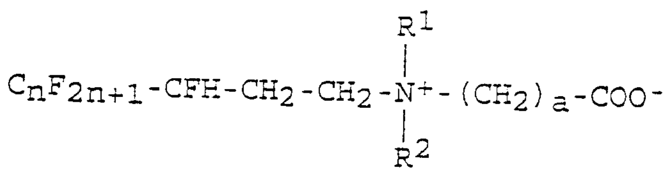

(2)

dans laquelle a vaut 1, 2, 3 ou 4, n est un nombre entier de 3 à 17 et $R^1$ et $R^2$ représentent alkyle en $C_1$-$C_4$, hydroxyalkyle en $C_1$-$C_4$ ou l'hydrogène, à la condition que seulement l'un des deux substituants $R^1$ et $R^2$ soit l'hydrogène.

**2.** Procédé pour la préparation de carboxyalkylbétaïnes selon la revendication 1, caractérisé en ce que l'on carboxyle des fluoralkylamines de formule (1) ci-après

$$\begin{array}{rl} & R^1 \\ & / \\ C_nF_{2n+1}-CFH-CH_2-CH_2-N & \\ & \backslash \\ & R^2 \end{array} \qquad (1)$$

dans laquelle n, $R^1$ et $R^2$ ont les significations données dans la revendication 1, avec un acide halogénocarboxylique de formule (2a) ci-après

$$X-(CH_2)_a-COOH \qquad (2a)$$

dans laquelle X est un halogène et a a la signification donnée, ou un sel de ces composés ou un ester d'alkyle en $C_1$-$C_4$, dans le cas de l'utilisation de l'ester d'acide halogénocarboxylique, on soumet le produit d'alkylation à une saponification.

3. Procédé pour la réduction de la tension superficielle des systèmes eau/air et de la tension de l'interface des systèmes eau/hydrocarbures, caractérisé en ce que l'on ajoute au système une quantité efficace de carboxyalkylbétaïnes de la revendication 1.

4. Alkylsulfobétaïnes de formule (3) ci-après

$$\begin{array}{c} R^1 \\ | \\ C_nF_{2n+1}-CFH-CH_2-CH_2-N-(CH_2)_b-SO_3^- \\ | \\ R^2 \end{array} \qquad (3)$$

dans laquelle b vaut 1, 2, 3 ou 4, n est un nombre entier de 3 à 17 et $R^1$ et $R^2$ représentent un alkyle en $C_1$-$C_4$, hydroxyalkyle en $C_1$-$C_4$ ou un atome d'hydrogène, à la condition que seulement l'un des deux substituants $R^1$, $R^2$ soit l'hydrogène.

5. Procédé pour la préparation des alkylsulfobétaïnes selon la revendication 4, caractérisé en ce que l'on sulfoalkyle des fluoralkylamines de formule (1) ci-après

$$\begin{array}{rl} & R^1 \\ & / \\ C_nF_{2n+1}-CFH-CH_2-CH_2-N & \\ & \backslash \\ & R^2 \end{array} \qquad (1)$$

dans laquelle n, $R^1$ et $R^2$ ont les significations données dans la revendication 4, avec une sulfone de formule (3a) ci-après

$$\begin{array}{c} (CH_2)_b \\ / \quad \backslash \\ O — SO_2 \end{array} \qquad (3a)$$

dans laquelle b a la signification donnée dans la revendication 4.

6. Procédé pour la réduction de la tension superficielle des systèmes eau/air et de la tension sur l'interface des systèmes eau/hydrocarbures, caractérisé en ce que l'on ajoute au système une quantité efficace d'alkylsulfobétaïnes selon la revendication 4.

**7.** Mélange de carboxyalkylbétaïnes constitué essentiellement

A) de 60 à 90 % en poids d'au moins une carboxyalkylbétaïne de formule (2) ci-après

$$C_nF_{2n+1}-CFH-CH_2-CH_2-\overset{R^1}{\underset{R^2}{N^+}}-(CH_2)_a-COO^- \qquad (2)$$

dans laquelle a vaut 1, 2, 3 ou 4, n est un entier de 3 à 17 et $R^1$ et $R^2$ représentent un groupe alkyle en $C_1$-$C_4$, hydroxyalkyle en $C_1$-$C_4$ ou un atome d'hydrogène, à la condition que seulement l'un des deux substituants $R^1$ et $R^2$ soit un atome d'hydrogène, et

B) de 10 à 40 % en poids d'au moins une carboxyalkylbétaïne de formule (5) ci-après

$$C_nF_{2n+1}-CH_2-CH_2-CH_2-\overset{R^1}{\underset{R^2}{N}}-(CH_2)_a-COO^- \qquad (5)$$

dans laquelle a, n, $R^1$ et $R^2$ ont les significations données,

les pourcentages en poids étant relatifs au poids du mélange.

**8.** Procédé pour la préparation de mélanges selon la revendication 7, caractérisé en ce que l'on soumet des mélanges de composés de fluoralkylamines constitués essentiellement

A') de 60 à 90 % en poids d'au moins une fluoralkylamine de formule (1) ci-après

$$C_nF_{2n+1}-CFH-CH_2-CH_2-N\begin{matrix} / R^1 \\ \backslash R^2 \end{matrix} \qquad (1)$$

dans laquelle n est un entier de 3 à 17 et $R^1$ et $R^2$ représentent un groupe alkyle en $C_1$-$C_4$, hydroxyalkyle en $C_1$-$C_4$ ou un atome d'hydrogène, à la condition que seulement l'un des deux substituants $R^1$ et $R^2$ soit un atome d'hydrogène, et

B') de 10 à 40 % en poids d'au moins une fluoralkylamine saturée de formule (4) ci-après

$$C_nF_{2n+1}-CH_2-CH_2-CH_2-N\begin{matrix} / R^1 \\ \backslash R^2 \end{matrix} \qquad (4)$$

dans laquelle n, $R^1$ et $R^2$ ont les significations données ci-dessus,

les pourcentages en poids étant relatifs au poids du mélange, à une carboxyalkylation avec un acide halogénocarboxylique de formule (2a) ci-après

$$X\text{-}(CH_2)_a\text{-}COOH \qquad (2a)$$

dans laquelle X est un atome d'halogène et a a la signification donnée,
ou un sel de celui-ci ou un ester d'alkyle en $C_1$-$C_4$, où dans le cas de l'utilisation de l'ester d'acide halogénocarboxylique, on soumet le produit d'alkylation à une saponification.

**9.** Procédé pour la réduction de la tension superficielle des systèmes eau/air et de la tension sur l'interface des systèmes eau/hydrocarbures, caractérisé en ce que l'on ajoute au système une quantité efficace du mélange de carboxyalkylbétaïne selon la revendication 7.

**10.** Mélanges de sulfobétaïnes constitués essentiellement

A) de 60 à 90 % en poids d'au moins une alkylsulfobétaïne de formule (3) ci-après

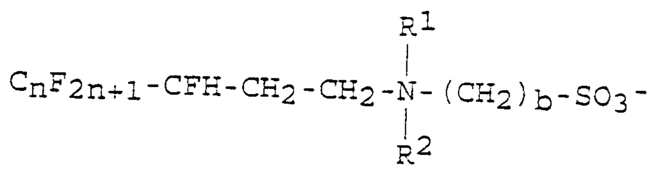
(3)

où b vaut 1, 2, 3 ou 4, n est un entier de 3 à 17, et $R^1$ et $R^2$ représentent un groupe alkyle en $C_1$-$C_4$, hydroxyalkyle en $C_1$-$C_4$ ou un atome d'hydrogène, à la condition que seulement l'un des deux substituants $R^1$ et $R^2$ soit un atome d'hydrogène, et
B) de 10 à 40 % en poids d'au moins une alkylsulfobétaïne de formule (6) ci-après

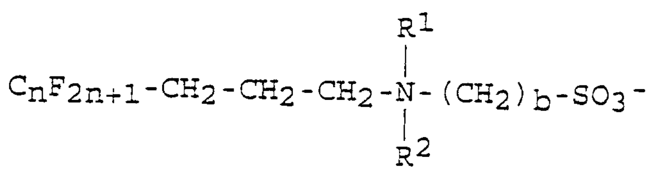
(6)

dans laquelle b, n, $R^1$ et $R^2$ ont les significations données ci-dessus,
les pourcentages en poids étant relatifs au poids du mélange.

**11.** Procédé pour la préparation de mélanges selon la revendication 10, caractérisé en ce que l'on sulfoalkyle des mélanges de composés de fluoralkylamines constitués essentiellement

A') de 60 à 90 % en poids d'au moins une fluoralkylamine saturée de formule (1) ci-après

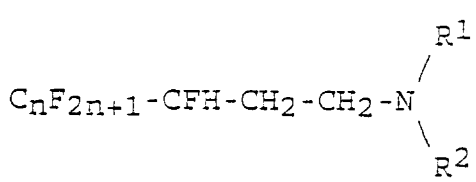
(1)

dans laquelle n est un entier de 3 à 17 et $R^1$ et $R^2$ représentent un groupe alkyle en $C_1$-$C_4$, hydroxyalkyle en $C_1$-$C_4$ ou un atome d'hydrogène, à la condition que seulement l'un des deux substituants $R^1$ et $R^2$ soit un atome d'hydrogène, et
B') de 10 à 40 % en poids d'au moins une fluoralkylamine saturée de formule (4) ci-après

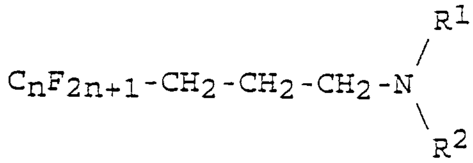

(4)

dans laquelle n, $R^1$ et $R^2$ ont les significations données ci-dessus, les pourcentages en poids étant relatifs au poids du mélange, avec une sultone de formule (3a) ci-après

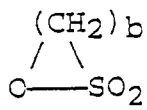

(3a)

dans laquelle b a la signification donnée.

**12.** Procédé pour la réduction de la tension superficielle des systèmes eau/air et de la tension sur l'interface des systèmes eau/hydrocarbures, caractérisé en ce que l'on ajoute au système une quantité efficace du mélange d'alkylsulfobétaïnes selon la revendication 10.